# FASCICULE DE BREVET EUROPEEN

(11) **EP 2 320 794 B1**
(45) Date de publication et mention de la délivrance du brevet: **04.06.2014**
(21) Numéro de dépôt: 09766286.0
(22) Date de dépôt: 18.06.2009
(51) Int. Cl.: A61B 5/103

(54) **DISPOSITIF DE MESURE DES PROPRIETES MECANIQUES D'UNE ZONE DE TISSU BIOLOGIQUE**
VORRICHTUNG ZUR MESSUNG DER MECHANISCHEN EIGENSCHAFTEN EINES BEREICHS VON BIOLOGISCHEM GEWEBE
DEVICE FOR MEASURING THE MECHANICAL PROPERTIES OF AN AREA OF BIOLOGICAL TISSUE

(30) Priorité: 20.06.2008 FR 0803458
(43) Date de publication de la demande: 18.05.2011
(73) Titulaire: Peritesco, 75001 Paris (FR)
(72) Inventeur: ZAHOUANI, Hassan, F-25000 Besançon (FR); BOYER, Gaëtan, 34380 MAS de LONDRES (FR); PERICOI, Marc, F-92410 Ville d'Avray (FR)
(74) Mandataire: Monlouis, Patrick
(86) Numéro de dépôt international: PCT/IB2009/052601
(87) Numéro de publication internationale: WO 2009/153749

(56) Documents cités:
- WO-A-2005/039379
- WO-A1-2009/144680
- FR-A- 2 833 827
- US-A- 5 372 030
- US-A- 6 042 544
- US-A1- 2005 068 544

## Description

La présente invention est relative à un dispositif de mesure des propriétés mécaniques d'une zone de tissu biologique comprenant une source de gaz permettant d'injecter suivant un axe Y-Y du gaz sous pression vers la zone de tissu et une source lumineuse émettant suivant l'axe Y-Y un faisceau lumineux vers la zone de tissu.

WO 03/020122 décrit un dispositif de mesure de la pression interne de l'oeil utilisant un faisceau lumineux et un système d'injection d'air.

Néanmoins ce système est destiné à mesurer la pression à l'intérieur de l'oeil et non des caractéristiques propres à la cornée.

De plus la cornée a une structure différente d'autres tissus biologiques, du fait notamment de son caractère transparent, ce qui ne permet pas de généraliser l'utilisation du dispositif selon WO 03/020122.

De nombreux traitements sont développés pour améliorer les caractéristiques de la peau. Cette évolution crée une demande pour des dispositifs capables de mesurer les caractéristiques de la peau avant et après traitement.

Par ailleurs, lors d'une opération, l'incision nécessaire de la peau va modifier localement les caractéristiques de la peau en créant une cicatrice. La gêne occasionnée pourrait être utilement quantifiée s'il était possible de mesurer l'évolution des caractéristiques de la peau à cet endroit.

Le dispositif selon l'invention a notamment pour but de répondre aux besoins mentionnés ci-dessus

Selon l'invention un dispositif de mesure des propriétés mécaniques d'une zone de tissu biologique du genre défini précédemment est caractérisé en ce que le dispositif est prévu pour réaliser des mesures sur la peau et comprend :
- un tube d'injection de gaz d'axe Y-Y, dont l'extrémité libre est destinée à être placée à proximité de la zone de peau à examiner, canalisant le jet de gaz,
- un capteur de lumière, décalé par rapport à l'axe Y-Y, destiné à recueillir une partie des rayons du faisceau renvoyés par la zone de peau.

Avantageusement, le faisceau lumineux utilisé est un faisceau laser. Par ailleurs, le faisceau lumineux peut avoir un diamètre compris entre 70 et 350 µm.

Le tube peut avoir un diamètre inférieur ou égal à 5 mm et en particulier de 2mm.

Le dispositif comprend un calculateur apte à déterminer par triangulation le déplacement de la zone de la peau au niveau du point d'impact du faisceau lumineux.

Avantageusement pour un débit de gaz donné, on mesure la déformation de la peau générée ou bien pour une déformation donnée, on mesure le débit de gaz utilisé.

La distance entre le capteur et la zone de la peau examinée peut être comprise entre 65 et 95 mm.

De manière avantageuse, le gaz utilisé est l'air.

D'autres caractéristiques et avantages de l'invention apparaîtront dans la description qui suit d'un mode de réalisation préféré avec référence aux dessins annexés mais qui n'a aucun caractère limitatif. Sur ces dessins :
Fig. 1 est une vue en perspective d'un dispositif selon l'invention.
Fig. 2 est une vue en coupe selon un plan II-II du dispositif de Fig. 1.
Fig. 3 est un graphique illustrant des mesures de déformation de la peau ainsi que la sollicitation mécanique utilisée en fonction du temps.
Fig. 4 est un détail de Fig. 2 illustrant plus particulièrement la réflexion sur la peau d'un faisceau émis par le dispositif de Fig. 1, et
Fig. 5 est une vue similaire à Fig. 4 illustrant la réflexion dudit faisceau avec et sans déformation de la peau.

Dans toute la description qui suit d'un mode de réalisation d'un dispositif de mesure des propriétés de la peau selon l'invention, les termes relatifs tels que « supérieur », « inférieur », « avant », « arrière », « horizontal » et « vertical » sont à interpréter lorsque le dispositif de mesure des propriétés de la peau est installé au dessus d'un patient, l'axe Y-Y, défini ci-après, étant vertical.

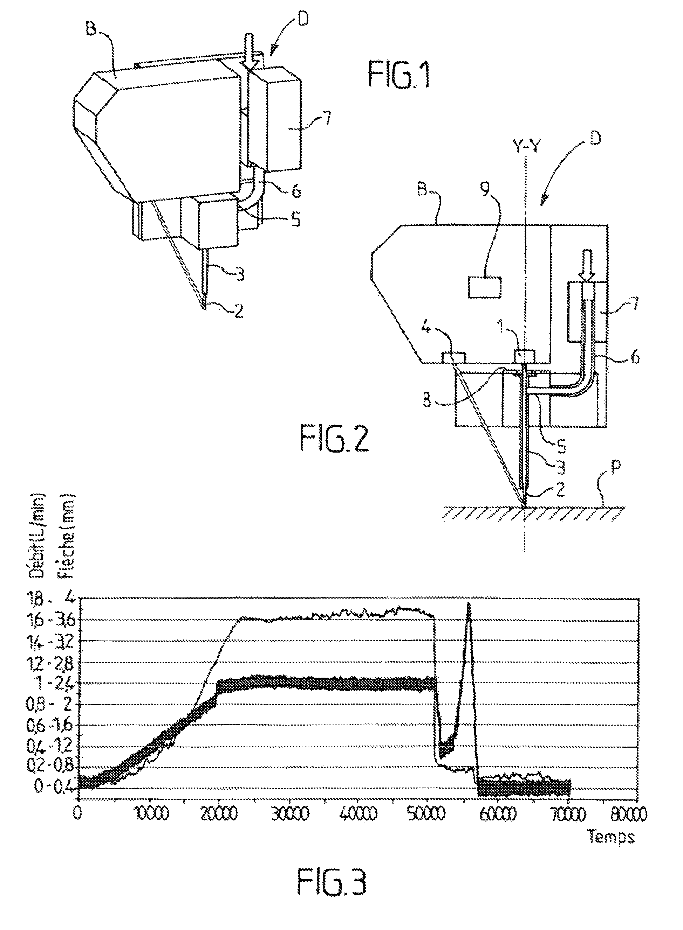

Comme on peut le voir sur Fig. 1 et Fig. 2, le dispositif D selon l'invention comprend un boîtier B dans lequel est mis en place une source laser 1 émettant un spot 2 selon un axe Y-Y. Le spot laser se prolonge selon l'axe Y-Y à l'intérieur d'un tube 3.

Lorsque le spot 2 rencontre un obstacle, par exemple la peau d'un patient, le spot est réfléchi et une partie des rayons réfléchis est détectée par un capteur 4. Le capteur 4 peut être un capteur CCD appelé détecteur à couplage de charge, mais aussi de type MOS ou CMOS.

Le tube 2 comporte un raccord 5 permettant une injection de gaz provenant d'une source de gaz S.

Le gaz utilisé de préférence est l'air. Le gaz est acheminé de la source S par un conduit 6 à travers une électrovanne 7.

La partie supérieure du tube 3 est fermée hermétiquement par une vitre transparente 8 située au dessus du raccord 5. De cette façon, le spot laser 2 émis par la source 1 peut passer dans le tube 3 tandis que le gaz venant de l'électrovanne 7 s'écoule vers l'extrémité inférieure du tube 3 et ne peut s'infiltrer au-delà de l'extrémité supérieure du tube 3 fermée par la vitre 8.

Un calculateur 9 est mis en place dans le boîtier B. Le calculateur 9 est capable à partir des informations fournies par la source 1 et le capteur 4 de déterminer par triangulation la position du point d'impact du spot 2 sur la peau P du patient.

En fonctionnement, l'injection de gaz sous pression à travers l'électrovanne 7 permet de solliciter mécaniquement une zone de la peau d'un patient située dans le prolongement du tube 3.

Cette sollicitation mécanique peut varier en fréquence et en intensité en régulant le débit du gaz.

L'étendue de la zone de la peau P sollicitée est définie par le diamètre du tube 3. De manière à permettre une mesure ponctuelle précise, un faible diamètre est préférable. On utilisera de préférence un tube d'un diamètre intérieur inférieur ou égal à 5 mm. Le mode de réalisation décrit utilise un tube 3 de 2 mm de diamètre intérieur.

Le fait de n'avoir qu'un point de mesure permet d'effectuer des mesures très rapides sans être obligé d'utiliser une caméra rapide, très coûteuse.

Le spot laser 2 est émis par la source 1 selon l'axe Y-Y et se propage vers le bas à l'intérieur du tube 3. Le diamètre d du spot laser varie de 70 µm à 350 µm.

Le spot 2 frappe la surface de la peau P du patient et est partiellement réfléchi. Une partie de la lumière réfléchie, et notamment un rayon rₘ, est détectée par le capteur 4.

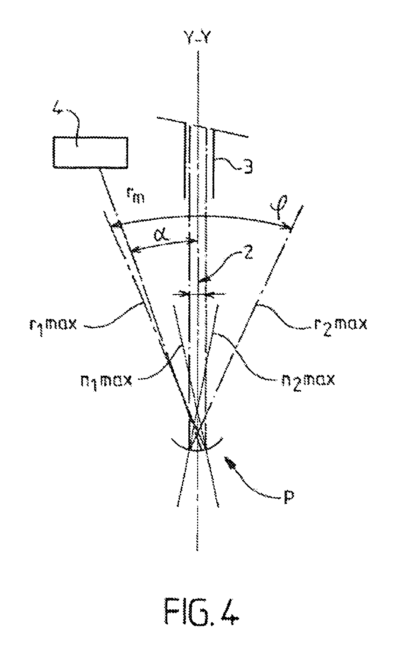

La réflexion du spot 2 sur la peau P est plus particulièrement illustrée Fig. 4.

Le spot 2 de diamètre d sortant du tube 3 est réfléchi en une multitude de rayons r. On distingue plus particulièrement les rayons r1ₘₐₓ et r2ₘₐₓ issus de la réflexion de rayons incidents frappant la peau P en des points ou les normales n1ₘₐₓ et n2ₘₐₓ forment un angle maximum avec l'axe Y-Y. Les rayons r1ₘₐₓ et r2ₘₐₓ permettent de définir un cône d'angle d'ouverture ϕ dans lequel se trouvent tous les rayons réfléchis issus du spot 2. Parmi ces rayons réfléchis, le rayon rₘ, formant un angle α avec l'axe Y-Y, est détecté par le capteur 4.

Le capteur 4, ou un dispositif associé, comporte une lentille E et un moyen de mesure de l'angle α. Le capteur 4 est de type linéaire, constitué d'un alignement de capteurs élémentaires ou bien constitué d'un agencement de capteurs élémentaires sous forme de matrice. Cette disposition permet de déterminer les coordonnées du point d'impact du rayon rₘ avec le capteur 4.

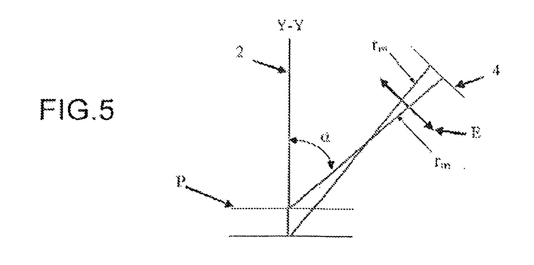

L'angle entre l'axe Y-Y du faisceau émis 2 et l'axe optique du système capteur 4 reste constant. On peut voir Fig. 5 que le rayon réfléchi va impacter la capteur 4 en des points différents en fonction du point d'impact du faisceau 2 sur la peau et donc de la déformation de celle-ci.

La mesure du déplacement observé sur le capteur 4 permet de déterminer la variation de l'angle α. La flèche mesurée est donc déduite à partir du calcul de l'angle α en connaissant les caractéristiques géométriques du système tel que la distance entre le capteur 4 et l'axe Y-Y.

L'impact de rayons incidents sur le capteur 4 permet de mesurer la déformation de la peau P suite à la sollicitation provoquée par le jet de gaz grâce à une analyse par triangulation dans un calculateur 9.

Utiliser la réflexion du spot 2 selon un axe différent de Y-Y facilite l'utilisation de la triangulation pour déterminer les coordonnées du point d'impact sur la peau.

La triangulation est réalisée de façon géométrique. Connaissant les paramètres géométriques du dispositif à savoir notamment l'angle α entre la source 1 et le capteur 4 et la distance entre les deux, on détermine la distance entre la source 1 et la peau P à l'aide de formules trigonométriques. L'utilisation de l'interférométrie est également possible mais plus complexe à mettre en oeuvre.

La sollicitation mécanique s'effectue dans l'axe de la mesure de la déformation. L'émission lumineuse, c'est-à-dire le spot 2, est envoyée dans l'axe de la sollicitation mécanique afin de s'assurer que le point mesuré se situe toujours au fond de la déformée. Une émission lumineuse avec un angle par rapport à l'axe de la sollicitation mécanique implique de mesurer non plus un point mais une surface pour être certain d'accéder à la flèche maximale

Un régulateur de débit de gaz (non représenté), en amont de l'électrovanne 7, permet de réguler le débit du gaz et de faire varier la fréquence et l'extrémité de la sollicitation mécanique.

On peut voir sur Fig. 3 un relevé, en fonction du temps, du débit gazeux (en gras) et de la flèche mesurée (en trait plus fin). Ceci illustre l'importance de la déformation de la peau P au regard de la sollicitation exercée. On constate la corrélation, au parasitage près, entre la déformation mesurée et la sollicitation exercée. Entre 0 et 10000 sur l'échelle de temps, la flèche et le débit croissent tout deux puis ils sont tous deux stables entre 10000 et 50000 pour chuter tous deux par la suite.

Ce dispositif permet la mesure à une distance de 65 à 95 mm du capteur 4.

Un diamètre du spot laser de 70 µm à 350 µm permet de détecter des déformations de la peau de 1,5 µm.

On utilise une fréquence de mesure pouvant aller jusqu'à 50 kHz mais de préférence de 10 kHz de manière à améliorer les performances.

Les sollicitations appliquées peuvent être variées, pouvant aller du fluage à un régime sinusoïdal, ce qui permet de mesurer les paramètres viscoélastiques intrinsèques à la peau en sus de la simple mesure de la pression intérieure du corps.

On notera en particulier la possibilité d'effectuer des mesures lors d'un cycle charge/décharge, de mesurer le débit de gaz pour une déformation donnée et de mesurer une déformation pour un débit de gaz donné.

Par ailleurs l'étendue de mesure ne se limite pas à un point. Le dispositif peut être utilisé sur l'ensemble du corps. Un bâti spécifique, non représenté, peut permettre au dispositif d'accéder à l'ensemble du corps et d'assurer une sollicitation mécanique perpendiculaire à la zone en contact.

## Revendications

1. Dispositif (D) de mesure des propriétés mécaniques d'une zone de tissu biologique comprenant une source de gaz permettant d'injecter suivant un axe Y-Y du gaz sous pression vers la zone de tissu et une source lumineuse (1) émettant suivant l'axe Y-Y un faisceau lumineux (2) vers la zone de tissu, où le dispositif (D) est prévu pour réaliser des mesures sur la peau (P) et comprend :
- un tube (3) d'injection de gaz d'axe Y-Y, dont l'extrémité libre est destinée à être placée à proximité de la zone de peau (P) à examiner, canalisant le jet de gaz,
- un capteur de lumière (4), décalé par rapport à l'axe Y-Y, destiné à recueillir une partie des rayons du faisceau (2) renvoyés par la zone de peau,
- un calculateur (9) apte à déterminer par triangulation le déplacement de la zone de la peau (P) au niveau du point d'impact du faisceau lumineux (2).

2. Dispositif (D) selon la revendication 1, **caractérisé en ce que** le faisceau lumineux (2) est un faisceau laser.

3. Dispositif (D) selon la revendication 1 ou 2, **caractérisé en ce que** le faisceau lumineux (2) a un diamètre compris entre 70 et 350 µm.

4. Dispositif (D) selon l'une quelconque des revendications précédentes, **caractérisé en ce que** le tube (3) a un diamètre inférieur ou égal à 5 mm.

5. Dispositif (D) selon la revendication 4, **caractérisé en ce que** le tube (3) a un diamètre de 2 mm.

6. Dispositif (D) selon la revendication 5, **caractérisé en ce que** pour un débit de gaz donné, on mesure la déformation de la peau (P) générée.

7. Dispositif (D) selon la revendication 5, **caractérisé en ce que** pour une déformation donnée, on mesure le débit de gaz utilisé.

8. Dispositif (D) selon l'une quelconque des revendications précédentes, **caractérisé en ce que** la distance entre le capteur (4) et la zone de la peau (P) examinée est comprise entre 65 et 95 mm.

9. Dispositif (D) selon l'une quelconque des revendications précédentes, **caractérisé en ce que** le gaz utilisé est l'air.

## Patentansprüche

1. Vorrichtung (D) zum Messen der mechanischen Eigenschaften einer Zone biologischen Gewebes, mit einer Gasquelle, die es ermöglicht, druckbeaufschlagtes Gas entlang einer Achse Y-Y in Richtung der Gewebezone zu injizieren, und einer Lichtquelle (1), welche ein Lichtbündel (2) entlang der Achse Y-Y in Richtung der Gewebezone emittiert, wobei die Vorrichtung (D) zur Durchführungen von Messungen an der Haut (P) vorgesehen ist und aufweist:
- ein den Gasstrahl kanalisierendes Gasinjektionsrohr (3) mit der Achse Y-Y, dessen freies Ende zur Platzierung nahe der zu untersuchenden Hautzone (P) vorgesehen ist,
- einen in Bezug auf die Achse Y-Y versetzten Lichtsensor (4) zum Erfassen eines Teils der von der Hautzone (P) reflektierten Strahlen des Lichtbündels (2),
- einen Rechner (9), der in der Lage ist, durch Triangulation die Verschiebung der Hautzone (P) an dem Auftreffpunkt des Lichtbündels (2) zu bestimmen.

2. Vorrichtung (D) nach Anspruch 1, **dadurch gekennzeichnet, dass** das Lichtbündel (2) ein Laserstrahl ist.

3. Vorrichtung (D) nach Anspruch 1 oder 2, **dadurch gekennzeichnet, dass** das Lichtbündel (2) einen Durchmesser zwischen 70 und 350 µm aufweist.

4. Vorrichtung nach einem der vorhergehenden Ansprüche, **dadurch gekennzeichnet, dass** das Rohr (3) einen Durchmesser aufweist, der kleiner oder gleich 5 mm ist.

5. Vorrichtung (D) nach Anspruch 4, **dadurch gekennzeichnet, dass** das Rohr (3) einen Durchmesser von 2 mm aufweist.

6. Vorrichtung (D) nach Anspruch 5, **dadurch gekennzeichnet, dass** die erzeugte Verformung der Haut (P) bei einem gegebenen Gasdurchsatz gemessen wird.

7. Vorrichtung (D) nach Anspruch 5, **dadurch gekennzeichnet, dass** der verwendete Gasdurchsatz bei einer gegebenen Verformung gemessen wird.

8. Vorrichtung (D) nach einem der vorhergehenden Ansprüche, **dadurch gekennzeichnet, dass** der Abstand zwischen den Sensor (4) und der untersuchten Hautzone (P) zwischen 65 und 95 mm beträgt.

9. Vorrichtung (D) nach einem der vorhergehenden Ansprüche, **dadurch gekennzeichnet, dass** das verwendete Gas Luft ist.

## Claims

1. A device (D) for measuring the mechanical properties of an area of biological tissue comprising a gas source for injecting pressurized gas according to an axis Y-Y toward the area of tissue and a light source (1) emitting a light beam (2) according to the axis Y-Y toward the area of tissue, where the device (D) is designed to perform measurements on the skin (P) and comprises:
- a tube (3) of axis Y-Y for injecting gas, the free end of which is intended to be placed in proximity to the area of skin (P) to be examined, channeling the gas jet,
- a light sensor (4), offset relative to the axis Y-Y, intended to collect a portion of the rays of the beam (2) returned by the area of skin,
- a computer (9) able to determine, by triangulation, the displacement of the area of the skin (P) at the point of impact of the light beam (2).

2. The device (D) as claimed in claim 1, **characterized in that** the light beam (2) is a laser beam.

3. The device (D) as claimed in claim 1 or 2, **characterized in that** the light beam (2) has a diameter of between 70 and 350 µm.

4. The device (D) as claimed in any one of the preceding claims, **characterized in that** the tube (3) has a diameter less than or equal to 5 mm.

5. The device (D) as claimed in claim 4, **characterized in that** the tube (3) has a diameter of 2 mm.

6. The device (D) as claimed in claim 5, **characterized in that**, for a given gas flow rate, the resulting deformation of the skin (P) is measured.

7. The device (D) as claimed in claim 5, **characterized in that**, for a given deformation, the gas flow rate used is measured.

8. The device (D) as claimed in any one of the preceding claims, **characterized in that** the distance between the sensor (4) and the area of the skin (P) being examined is between 65 and 95 mm.

9. The device (D) as claimed in any one of the preceding claims, **characterized in that** the gas used is air.
